# EUROPÄISCHE PATENTSCHRIFT

(11) **EP 0 473 625 B1**
(45) Veröffentlichungstag und Bekanntmachung des Hinweises auf die Patenterteilung: **18.08.1993**
(21) Anmeldenummer: 90907022.9
(22) Anmeldetag: 17.05.1990
(51) Int. Cl.: G05D 21/02

(54) **EINRICHTUNG ZUM DOSIEREN VON DESINFEKTIONSMITTELN**
DEVICE FOR DISPENSING DISINFECTANTS
DISPOSITIF DE DOSAGE POUR PRODUITS DESINFECTANTS

(30) Priorität: 24.05.1989 DE 3916910
(43) Veröffentlichungstag der Anmeldung: 11.03.1992
(73) Patentinhaber: Henkel Kommanditgesellschaft auf Aktien, 40191 Düsseldorf (DE)
(72) Erfinder: WERNER-BUSSE, Alfred, D-4000 Düsseldorf 13 (DE); KLING, Hans-Willi, D-5600 Wuppertal 2 (DE); KUHLMANN, Werner, D-4019 Monheim 2 (DE); VOSS, Meike, D-4000 Düsseldorf (DE)
(86) Internationale Anmeldenummer: EP9000794
(87) Internationale Veröffentlichungsnummer: WO9014623

(56) Entgegenhaltungen:
- EP-A- 65 209
- EP-A- 231 843
- EP-A- 239 468
- EP-A- 260 187
- DE-A- 3 216 527
- DE-A- 3 400 263
- DE-A- 3 713 238
- FR-A- 2 243 729
- FR-A- 2 578 988

## Beschreibung

Die Erfindung betrifft eine Dosiereinrichtung von Desinfektionsmittel in Waschflotten mit einem Dosierer, einer insbesondere kontinuierlich betreibbaren Analysezelle in einer Abzweigung der Strömung der Waschflotte zum Erfassen des Gehalts an nichtverbrauchtem Desinfektionsmittel in der Waschflotte, mit einer an die Analysezelle und den Dosierer angeschlossenen Vergleichseinrichtung zum Vergleichen des vom Meßsystem abgegebenen Istwerts mit einem vorgegebenem Sollwert. Der Dosierer wird von dieser Vergleichseinrichtung beim Unterschreiten des Istwerts unter den Sollwert betätigt. Dem Einlaßbereich der Analysenzelle ist ein Probenaufbereitungsteil vorgeschaltet. Eine solche Einrichtung ist aus EP-A1-0 239 468 bekannt.

In Krankenhäusern und Heimen fällt infektiöse bzw. infektionsverdächtige Wäsche an, die desinfizierend gewaschen bzw. desinfiziert werden muß. Dies geschieht üblicherweise in gewerblichen oder institutionellen Wäschereien in der Regel durch ein desinfizierendes Waschverfahren, in dem ein Waschmittel und ein Desinfektionsmittel eingesetzt werden. Zu den Desinfektionsmitteln gehören insbesondere solche auf Peressigsäure- und Wasserstoffperoxidbasis wie z. B. Ozonit^{R} (eingetragenes Warenzeichen).

Die auf das Wäschgewicht bezogene einzusetzende Menge des Desinfektionsmittels berücksichtigt, daß sowohl durch das eingesetzte Waschmittel als auch durch die organische Belastung der Waschflotte, nämlich Schmutz, Blut und Mikroorganismen im weitesten Sinne, ein Verbrauch des Desinfektionsmittels, d. h. im Fall von oxidierenden Desinfektionsmitteln eine Sauerstoffzehrung eintritt. Die Dosierung des Desinfektionsmittels muß daher eine gewisse Reserve enthalten, die in Phasen von Spitzenbelastungen gerade ausreicht, jedoch in sonstigen Phasen eher eine großzügige Dosierung darstellt. Daher sind Spitzenbelastungen an Blut und Mikroorganismen mit einem gewissen Restrisiko verbunden, während bei geringeren Belastungen unnötig viel Desinfektionsmittel dosiert wird.

In FR-A-2 578 988 wird ein Regelsystem zur Konzentration von Desinfektionslösungen beschrieben, bei der Messungen des pH-Wertes, der Leitfähigkeit und des Redoxpotentials der Lösung verwendet wird. Aus FR-A-2 243 729 bzw. der dazu korrespondierenden DE-A-2 444 199 ist eine weitere Vorrichtung zum Regeln der Konzentration von chemischen Flüssigkeiten, z. B. des Chlorgehaltes von Schwimmbädern, bekannt. Die in EP-A2-0 260 187 genannte Einrichtung zum Behandeln von Abwasser mit Sauerstoff verwendet zum Erfassen des Sauerstoffgehaltes einen Sensor. Bei der in DE-A1-3 400 263 beschriebenen Einrichtung wird ebenfalls wie in der vorliegenden Anmeldung die Konzentration von Desinfektionsmittel in anzuwendenden Lösungen dieses Desinfektionsmittels erfaßt und überwacht, wobei jedoch eine Leitfähigkeitsmessung zum Bestimmen frisch hergestellter Lösungen verwendet wird. Dabei werden keine trübe Flüssigkeiten verwendet, so daß sich die oben genannten Probleme nicht stellen. Eine Probenaufbereitung unter Verwendung einer Dialysemembraneinheit ist aus den genannten Dokumenten nicht bekannt.

Die in DE-A1-3 216 527 beschriebene Dialysevorrichtung enthält eine Dialysemembran, die bestimmungsgemäß zur Trennung eines Blutstroms von einer Dialysierlösung eingesetzt wird. Bei der Zubereitung der Dialysierlösung wird deren Konzentration mittels einer Leitfähigkeits-Meßstelle gemessen. Eine Verwendung der Membran zur Aufbereitung einer Meßprobe ist dem Dokument nicht zu entnehmen.

In der vorliegenden Erfindung soll die Aufgabe gelöst werden, mit einer Dosiereinrichtung belastungsabhängig die richtige Desinfektionsmittelmenge zu dosieren, um in allen Fällen eine ausreichend sichere, aber nicht unnötig hohe Desinfektionsmittelmenge zur Verfügung zu stellen. Ein zu hoher Verbrauch des Desinfektionsmittels während des Desinfiziervorgangs soll erfaßt werden, damit nachdosiert werden kann.

Diese Aufgabe wird dadurch gelöst, daß die Analysenzelle eine photometrische oder nepholometrische Zelle ist, mit der die bei der Reaktion des nichtverbrauchten Desinfektionsmittels mit einem entsprechenden Reagenz auftretende Farbänderung bzw. Trübung meßbar ist, und daß der Probenaufbereitungsteil eine Dialysemembraneinheit als Rückhalteeinrichtung für feste Stoffe aufweist.

Die bei der Reaktion des nichtverbrauchten Desinfektionsmittels mit einem entsprechenden Reagenz auftretenden Farbänderung bzw. Trübung wird mit der photometrischen oder nepholometrischen Zelle gemessen. Die über die Abzweigung der Analysenzelle zugeführte desinfizierende Waschflotte wird in der Analysenzelle mit den Reagenzien umgesetzt, so daß in der Reagenzlösung eine Farbänderung bzw. Trübung eintritt, die photometrisch bzw. nepholometrisch erfaßt und mit einem bestimmten Sollwert verglichen wird. Dieser Sollwert ist vorgegeben durch die gesetzlich festgelegte Mindestmenge an Desinfektionsmittel.

Die erfindungsgemäße Einrichtung erfaßt insbesondere kontinuierlich den Gehalt an nichtverbrauchtem Desinfektionsmittel und dosiert gegebenenfalls automatisch nach, bis daß der Sollwert wieder eingestellt ist.

Damit bei einer nicht kontinuierlichen Dosierung die Desinfektion durch rechtzeitige Nachdosierung sichergestellt ist, wird vorgeschlagen, daß die Einrichtung taktgesteuert ist und die Taktzeit ausreichend kurz zum Sicherstellen des Mindestgehalts von Desinfektionsmitteln in,der Waschflotte ist. Eine solche Einrichtung ist besonders vorteilhaft bei kontinuierlichen Waschstraßen.

Insbesondere wird vorgeschlagen, daß die Taktzeit zwischen 20 Sekunden und 5 Minuten liegt.

Bei einer erfindungsgemäßen Einrichtung zum Dosieren von oxidierenden Desinfektionsmitteln, insbesondere Percarbonsäure und/oder Wasserstoffperoxid wird weiterhin vorgeschlagen, daß die Analysenzelle ein Reagenz enthält, das mit dem Desinfektionsmittel ein Redoxsystem bildet. Insbesondere sind für eine photometrisch auswertbare Reaktion folgende Peroxo-Redoxsysteme vorteilhaft, z. B. das System Titanyl/Peroxotitanylcation, Chromionen der Oxidationsstufen 3 und 6, Vanadinsalze der Oxidationsstufen 3 und 5, Jodid/Jodsysteme, Mangan(II)-Permanganatsysteme und organische Redoxsysteme, z. B. 8-Hydroxychinolin/Oxychinolinverbindungen. In allen diesen Fällen tritt eine Farbänderung bzw. Trübungsänderung ein, deren vom Photometer erfaßten Extinktionswerte mit den Sollwerten verglichen werden, die bei desinfektionsgerechter Dosierung erreicht werden. Bei Unterschreiten dieses Sollwerts wird ein Impuls zur Nachdosierung ausgelöst. Das System dosiert in diesem Fall so lange Desinfektionsmittel nach, bis durch die kontinuierliche analytische Erfassung der Sauerstoffkonzentration die erreichte Sollmenge analysiert wird und der Dosierer dadurch wieder ausgeschaltet wird. Alternativ kann die nachzudosierende Menge berechnet werden, die durch den Dosierer der Waschflotte zugegeben wird. Dieser Meßvorgang kann sich alle 30 Sekunden wiederholen. Es wird also insbesondere vorgeschlagen, daß das Reagenz die zu oxidierende Komponente des genannten Redoxsystems bildet.

Vorteilhaft ist außerdem, wenn die Rückhalteeinrichtung einen Filter aufweist.

Ferner wird vorgeschlagen, daß die Vergleichseinrichtung als Mikroprozessor ausgebildet ist.

Die erfindungsgemäße Einrichtung kann separat und unabhängig von einer Waschmitteldosieranlage angeordnet sein, aber eine in eine Waschmitteldosieranlage integrierte Einrichtung ist ebenfalls möglich.

Im folgenden wird ein Ausführungsbeispiel der Erfindung anhand der Zeichnungen naher erläutert. Es zeigen
- Figur 1: ein Schema einer Waschmitteldosieranlage mit der erfindungsgemäßen Einrichtung zum Dosieren von Desinfektionsmitteln,
- Figur 2: eine schematische Darstellung des Probenaufbereitungsteils in Figur 1 und
- Figur 3: den Verlauf der Desinfektionsmittelkonzentration während des Waschprozesses bei einem Einsatz der erfindungsgemäßen Einrichtung zum Dosieren von Desinfektionsmitteln.

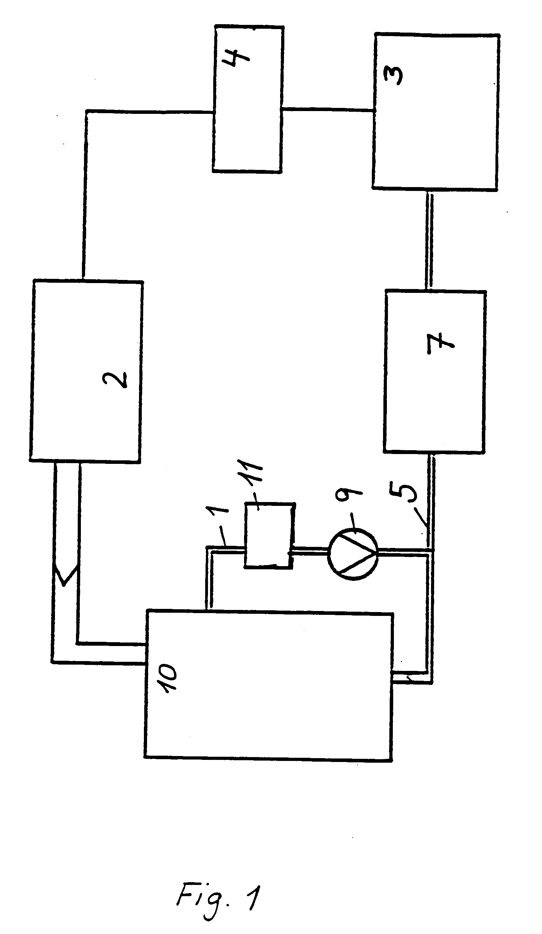

Gemäß Figur 1 wird eine Waschflotte 1 von einer Pumpe 9 aus einer gewerblichen Waschmaschine 10 über einen Filter 11 wieder in diese Waschmaschine 10 zurückgepumpt. Desinfektionsmittel wird von einem Dosierer 2 in die gewerbliche Waschmaschine 10 zugegeben. Der Dosierer 2 wird von einer als Mikroprozessor ausgebildeten Vergleichseinrichtung 4 angesteuert, die ihre Signale von einem eine Analysenzelle aufweisenden Meßsystem 3 erhält. Die vom Meßsystem 3 zu analysierende Flüssigkeit wird von der Waschflotte 1 abgezweigt und läuft durch die Abzweigung 5 über einen Probenaufbereitungsteil 7 zur Analysenzelle 3.

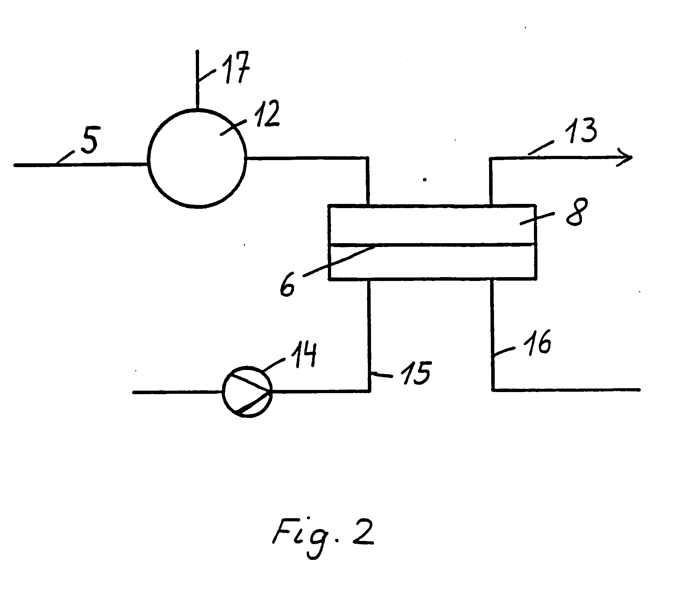

Dieser Probenaufbereitungsteil 7 ist in der vorliegenden Erfindung besonders wichtig und wird in Figur 2 ausführlicher dargestellt. Um sichere und reproduzierbare Ergebnisse zu erhalten und auf Grund solcher Ergebnisse den Dosierer 2 ansteuern zu können, ist es nämlich notwendig, daß die in die Analysenzelle 3 fließende Flüssigkeit von den groben Verunreinigungen der Waschflotte 1 befreit wird. Von der Abzweigung 5 läuft die Waschflotte über ein Mehrwegeventil 12 zu einer Dialyseeinheit 8 und von dort aus in die Abwasserleitung 13. Eine Reagenzlösung wird von einer weiteren Pumpe 14 durch die Reagenzleitung 15 zur Dialyseeinheit 8 gepumpt. In dieser Dialyseeinheit 8 fließt die Reagenzlösung auf der anderen Seite der Dialysemembran 6, die der Waschflotte gegenüber liegt, nimmt auf diese Weise flüssige Bestandteile aus der Waschflotte auf und reagiert mit diesen. Die Reagenzflüssigkeit fließt dann über die Leitung 16 weiter zum Analysator. Um die Einrichtung zum Dosieren von Desinfektionsmitteln zu eichen, können über das Mehrwegeventil 12 Standardlösungen über die Leitung 17 in die Dialyseeinheit 8 eingebracht werden.

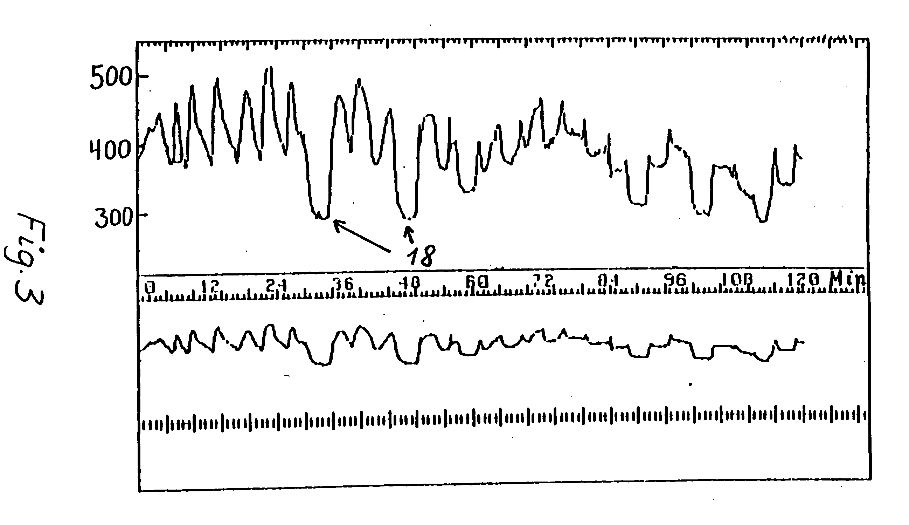

Figur 3 zeigt schließlich den Verlauf der Desinfektionsmittelkonzentration während des Waschprozesses. Dabei gibt die obere Kurve den Gehalt an Aktivsauerstoff in der Waschflotte in Einheiten ppm an. Die beiden Pfeile 18 weisen auf eine künstliche Störung der Dosierung hin, die zu Testzwecken durchgeführt wurde. Die untere Kurve stellt das Signal am Ausgang der Vergleichseinrichtung 4 dar. Es ist deutlich zu erkennen, daß das Signal der Vergleichseinrichtung 4 den Gehalt an Aktivsauerstoff in der Waschflotte gut repräsentiert.

### Bezugszeichenliste

- 1: Waschflotte
- 2: Dosierer
- 3: Meßsystem, Analysenzelle
- 4: Vergleichseinrichtung, Mikroprozessor
- 5: Abzweigung
- 6: Dialysemembran
- 7: Probenaufbereitungsteil
- 8: Rückhalteeinrichtung, Dialyseeinheit
- 9: Pumpe
- 10: gewerbliche Waschmaschine
- 11: Filter
- 12: Mehrwegeventil
- 13: Abwasserleitung
- 14: Pumpe
- 15: Reagenzleitung
- 16: Leitung
- 17: Leitung

## Patentansprüche

1. Dosiereinrichtung von Desinfektionsmittel in Waschflotten (1) mit einem Dosierer (2), einer insbesondere kontinuierlich betreibbaren Analysezelle (3) in einer Abzweigung (5) der Strömung der Waschflotte (1) zum Erfassen des Gehalts an nichtverbrauchtem Desinfektionsmittel in der Waschflotte (1), mit einer an die Analysezelle (3) und den Dosierer (2) angeschlossenen Vergleichseinrichtung (4) zum Vergleichen des vom Meßsystem abgegebenen Istwerts mit einem vorgegebenem Sollwert, wobei der Dosierer (2) von dieser Vergleichseinrichtung (4) beim Unterschreiten des Istwerts unter den Sollwert betätigt wird, und mit einem dem Einlaßbereich der Analysenzelle vorgeschalteten Probenaufbereitungsteil (7),
**dadurch gekennzeichnet,**
daß die Analysenzelle (3) eine photometrische oder nepholometrische Zelle ist, mit der die bei der Reaktion des nichtverbrauchten Desinfektionsmittels mit einem entsprechenden Reagenz auftretende Farbänderung bzw, Trübung meßbar ist, und daß der Probenaufbereitungsteil (7) eine Dialysemembraneinheit (8) als Rückhalteeinrichtung für feste Stoffe aufweist.

2. Einrichtung nach Anspruch 1,
**dadurch gekennzeichnet,**
daß die Einrichtung taktgesteuert und die Taktzeit ausreichend kurz zum Sicherstellen des Mindestgehalts von Desinfektionsmitteln in der Waschflotte (1) ist.

3. Einrichtung nach Anspruch 2,
**dadurch gekennzeichnet,**
daß die Taktzeit zwischen 20 Sekunden und 5 Minuten liegt.

4. Einrichtung zum Dosieren von oxidierenden Desinfektionsmitteln, insbesondere Percarbonsäure und/oder Wasserstoffperoxid, nach einem der Ansprüche 1 bis 3,
**dadurch gekennzeichnet,**
daß die Analysenzelle (3) ein Reagenz enthält, das mit dem Desinfektionsmittel ein Redoxsystem bildet.

5. Einrichtung nach Anspruch 4,
**dadurch gekennzeichnet,**
daß das Reagenz die zu oxidierende Komponente des Redoxsystems bildet.

6. Einrichtung nach einem der Ansprüche 1 bis 5,
**dadurch gekennzeichnet,**
daß die Rückhalteeinrichtung (8) einen Filter aufweist.

7. Einrichtung nach einem der Ansprüche 1 bis 6,
**dadurch gekennzeichnet,**
daß die Vergleichseinrichtung als Mikroprozessor (4) ausgebildet ist.

## Claims

1. An arrangement for dosing disinfectants in wash liquors (1) comprising a doser (2), an analysis cell (3) - designed in particular for continuous operation - in a branch (5) of the flow of wash liquor (1) for determining the content of unused disinfectant in the wash liquor (1), a comparator (4) connected to the analysis cell (3) and the doser (2) for comparing the actual value provided by the measuring system with a predetermined set value, the doser (2) being designed for actuation by the comparator (4) whenever the actual value is below the set value, and a sample preparation section (7) preceding the entry zone of the analysis cell, characterized in that the analysis cell (3) is a photometric or nepholometric cell with which the change in color or colouding that occurs on reaction of the unused disinfectant with a corresponding reagent can be measured and in that the sample preparation section (7) comprises a dialysis membrane unit (8) as a retaining unit for solids.

2. An arrangement as claimed in claim 1, characterized in that it is controlled by a timer and the cycle time is sufficiently brief to ensure the minimum content of disinfectants in the wash liquor (1).

3. An arrangement as claimed in claim 2, characterized in that the cycle time is between 20 seconds and 5 minutes.

4. An arrangement for dosing oxidizing disinfectants, more particularly percarboxylic acid and/or hydrogen peroxide, as claimed in any of claims 1 to 3, characterized in that the analysis cell (3) contains a reagent which forms a redox system with the disinfectant.

5. An arrangement as claimed in claim 4, characterized in that the reagent forms the component of the redox system which is to be oxidized.

6. An arrangement as claimed in any of claims 1 to 5, characterized in that the retaining unit (8) comprises a filter.

7. An arrangement as claimed in any of claims 1 to 6, characterized in that the comparator is a microprocessor (4).

## Revendications

1. Dispositif de dosage d'agent désinfectant dans les bains de lavage (1), comprenant un doseur (2), une cellule d'analyse (3) actionnable en particulier en continu dans une dérivation (5) du courant du bain de lavage (1) pour saisir le contenu en agent désinfectant non utilisé dans le bain de lavage (1), un dispositif de comparaison (4) relié à la cellule d'analyse (3) et au doseur (2), afin de comparer la valeur effective transmise par le système de mesure avec une valeur théorique prédéfinie, le doseur (2) étant actionné par ce dispositif de comparaison (4) lorsque la valeur effective dévient inférieure à la valeur théorique, et un organe de préparation des échantillons installé en amont de la zone d'admission de la cellule d'analyse, caractérisé en ce que la cellule d'analyse (3) est une cellule photométrique ou néphélémétrique, qui permet de mesurer le changement de teinte ou le trouble se produisant lors de la réaction de l'agent désinfectant non utilisé avec un réactif correspondant et en ce que l'organe de préparation des échantillons (7) est une unité à membrane de dialyse (8) comme dispositif de retenue pour les matières solides.

2. Dispositif selon la revendication 1, caractérisé en ce qu'il est commandé par rythmeur et en ce que la durée du cycle est suffisamment brève pour garantir la concentration minimale en agent désinfectant dans le bain de lavage (1).

3. Dispositif selon la revendication 2, caractérisé en ce que la durée du cycle est comprise entre 20 secondes et 5 minutes.

4. Dispositif de dosage des agents désinfectants oxydants, en particulier l'acide percarboxylique et/ou l'eau oxygénée, selon l'une des revendications 1 à 3, caractérisé en ce que la cellule d'analyse (3) contient un réactif, qui forme un système redox avec l'agent désinfectant.

5. Dispositif selon la revendication 4, caractérisé en ce que le réactif forme le composant à oxyder du système redox.

6. Dispositif selon l'une des revendications 1 à 5, caractérisé en que le dispositif de retenue (8) présente un filtre.

7. Dispositif selon l'une des revendications 1 à 6, caractérisé en ce que le dispositif de comparaison est configuré comme un microprocesseur (4).
